(19) Europäisches Patentamt
European Patent Office
Office européen des brevets

(11) EP 0 734 419 B1

(12) EUROPEAN PATENT SPECIFICATION

(45) Date of publication and mention
of the grant of the patent:
**27.02.2002 Bulletin 2002/09**

(51) Int Cl.7: **C08L 67/02**, C08L 77/00,
C08L 75/04, C08L 23/06,
C08L 23/08, C08L 71/00

(21) Application number: **95905373.7**

(22) Date of filing: **13.12.1994**

(86) International application number:
**PCT/US94/14365**

(87) International publication number:
**WO 95/16746 (22.06.1995 Gazette 1995/26)**

(54) **BREATHABLE FILM**

ATMUNGSFÄHIGER FILM

FILMS PERMEABLES A L'AIR

(84) Designated Contracting States:
**BE DE FR GB IT NL**

(30) Priority: **13.12.1993 EP 93120042**

(43) Date of publication of application:
**02.10.1996 Bulletin 1996/40**

(73) Proprietor: **E.I. DU PONT DE NEMOURS AND
COMPANY
Wilmington Delaware 19898 (US)**

(72) Inventors:
• **CARDINAL, Jean-Claude
CH-1297 Founex (CH)**

• **HAUSMANN, Karl-Heinz
CH-2000 Neuchâtel (CH)**

(74) Representative:
**James, Anthony Christopher W.P.
Carpmaels & Ransford 43 Bloomsbury Square
London WC1A 2RA (GB)**

(56) References cited:
**JP-A- 56 100 846        JP-A- 59 221 353
JP-A- 60 058 447        US-A- 3 023 192
US-A- 4 368 295        US-A- 4 739 012**

## Description

This invention relates to a breathable film formed from a thermoplastic composition demonstrating moisture vapor, oxygen and carbon dioxide permeability, while acting as barriers to liquids and microorganisms such as viruses and bacteria. Such films are found in various articles including wound coverings, transdermal patches, operating room drapes, protective clothing, diapers, personal hygiene products (feminine hygiene, incontinency), waterproof and outdoor clothing articles, food packaging such as for fresh vegetables, fruit, cheese and meat, films used in plant growing environments and any end-use where it is desirable to combine "breathability" and liquid barrier properties to liquids and microorganisms.

Films made from the compositions according to the invention also demonstrate good adhesion to substrates made from materials containing certain functional groups that react with the "reactive groups" described hereinafter. Such substrates include melamine.

Background of the Invention

Various thermoplastic materials are known for forming films demonstrating both liquid barrier properties and moisture vapor permeability. Included among these are copolyether esters, copolyether amides and polyurethanes. While films of these materials perform well and meet the performance requirements of various end uses, a drawback they possess is their relative high cost in comparison to other thermoplastic materials.

It is an object of the present invention to provide a new breathable film, which is formed from a thermoplastic composition, possessing desirable performance characteristics at a reduced cost. Accordingly, the present invention provides a breathable film which is formed from a thermoplastic composition comprising:

(a) at least 25% by weight of a thermoplastic polymer selected from the group consisting of a block copolyether ester, a block copolyether amide, a polyurethane, or combinations thereof,
(b) from 10% to 70% by weight of a thermoplastic homopolymer of an alpha-olefin, a co- or terpolymer containing an alpha-olefin and one or more other monomers, or a block copolymer of a vinylarene and a conjugated diene with the provision that said component (b) is incompatible with (a); and
(c) from 0.1% to 15% by weight of a compatibilizer which is a homo- co- or terpolymer having a backbone which is identical to component (b) and a reactive group which is compatible with component (a)

wherein

(i) said film is obtained by blown film extrusion; and
(ii) said film has a moisture vapor transmission rate (MVTR) value, as measured according to ASTM E 96 BW which is approaching that of the theoretical MVTR value calculated as follows:

$$MVTR_{theor} = \left(MVTR_{comp.(a)}\right)\left(weight\%_{comp.(a)}\right) + \left(MVTR_{comp.(b)}\right)\left(weight\%_{comp.(b)}\right).$$

Component (b) of the composition is a lower cost thermoplastic material which has insufficient moisture vapor permeability to be suitable for the end-uses previously mentioned. It is incompatible with component (a) and, were the two to be combined together and formed into a film, such film would have a profile showing long laminae or sheets of component (b). Accordingly, films made from such a composition would have a permeability restricted by the specific permeability of component (b), and poor mechanical properties, e.g. a tendency to delaminate.

According to the present invention, it has been surprisingly found that a compatibilizer can be combined with components (a) and (b) to yield a film having a moisture vapor transmission rate (MVTR) approaching that of the theoretical value calculated as follows:

$$MVTR_{theor} = \left(MVTR_{comp.(a)}\right)\left(weight\%_{comp.(a)}\right) + \left(MVTR_{comp.(b)}\right)\left(weight\%_{comp.(b)}\right).$$

Another unexpected effect of the addition of the compatibilizer is the improved sealability and adhesion of the compatibilized blend to itself and other substrates, such as melamine.

The present invention furthermore provides a process for forming the breathable thermoplastic film of the invention comprising:

(a) preparing the thermoplastic composition comprising a mixture of (a), (b) and (c); and
(b) extruding said composition in a blown film extruder.

Detailed Description of the Invention

As used herein, certain terms are defined as follows.

"Compatibility" of thermoplastic materials is an art-recognized term that refers, generally, to the degree in which the thermoplastic materials are miscible and/or interact with each other. Accordingly, "incompatible", as used herein, means that the components (a) and (b) are substantially immiscible and/or do interact with each other.

"Compatibilizer" means a thermoplastic material which serves to prevent formation of the previously mentioned laminae of component (b) in a film according to the invention. The compatibilizer has a character which makes it simultaneously soluble or reactive with component (a) and interactive with component (b), thereby reducing the surface energy of component (b) in component (a), leading to the formation of a dispersion of globules of component (b) in the film.

Block copolyether esters, block copolyether amides and polyurethanes to be used for component (a) are known per se. Preferred block copolyether esters are segmented elastomers having soft polyether segments and hard polyester segments (c.f. U.S. Patent No. 4,739,012), available from the DuPont Company under the name Hytrel ®.

Suitable block copolyether amides for use in the invention are available under the name Pebax ® from Elf Atochem.

The amount of component (a) in the composition will vary depending upon the type of component (a), the type of component (b), desired level of moisture vapor permeability and other factors known to one skilled in the art. Component (a) is typically present in the composition according to the invention in an amount ranging from 25 to 90 % by weight, more preferably 50 to 80 %.

Component (b) according to the invention is, as previously mentioned, substantially incompatible with component (a). Component (b) may be a homopolymer of an alpha-olefin; a co- or terpolymer containing an alpha-olefin and one or more other monomers; or a block copolymer of a vinylarene and a conjugated diene.

Where component (b) is a homopolymer, it preferably contains the repeating unit $R-CH=CH_2$ in which R is hydrogen or an alkyl radical having between 1 and 8 carbon atoms. Preferred homopolymers according to the invention are polyethylene (low density (PE-LD), linear low density (PE-LLD), high density (HDPE) and very low density (VLDPE)) and polypropylene.

Where component (b) is a co- or terpolymer, it preferably contains the repeating unit $R-CH=CH_2$ above, with at least one further monoethylenically unsaturated, such as aliphatic or aromatic, monomer, the following of which can be cited by way of example: vinyl acetate, styrene, and (meth)acrylic derivatives. This other monomer can represent up to 20% by weight of the olefinic copolymer, preferably from 1 to 10% by weight.

Preferred copolymers to be used as component (b) according to the invention are copolymers of ethylene and propylene, ethylene-vinyl acetate copolymers, copolymers of ethylene and acrylic derivatives (e.g. copolymers of ethylene, carbon monoxide and n-butyl acrylate, commonly known as EnBACO), copolymers of ethylenically unsaturated carboxylic acid monomers (e.g. acrylic acid, methacrylic acid, crotonic acid, etc.) or the neutralized metallic salts thereof (e.g. as found in the partially neutralized ethylene/carboxylic acid copolymers which are commonly referred to in the art as ionomers), terpolymers based on olefin, methyl acrylate and ethyl acrylate or even mixtures of straight-chain and radicalar low density polyolefins.

Where component (b) is a block copolymer of a vinylarene and a conjugated diene, it may have the general structure A-B-A wherein the two terminal polymer blocks A comprise thermoplastic polymer blocks of vinylarenes such as polystyrene, while block B is a polymer block of selectively hydrogenated conjugated diene such as isoprene or butadiene (cf. U.S. Patent Re. No. 27,145; U.S. Patent No. 4,548,988). The proportion of the thermoplastic terminal blocks to the center elastomeric polymer block and the relative molecular weights of each of these blocks is balanced to obtain a rubber having an optimum combination of properties such that it behaves as a vulcanized rubber without requiring the actual step of vulcanization. Moreover, these block copolymers can be designed not only with this important advantage but also so as to be handled in thermoplastic forming equipment and are soluble in a variety of relatively low cost solvents.

Optionally, these block copolymers can be grafted with maleic anhydride so as to form adducts which contain 0.1 to 10% by weight, preferably 0.2 to 5%, of maleic anhydride (see U.S. Patent 4,578,429). Such compounds are commonly referred to as S-EB-S block copolymers and are available from Shell Chemical Company under the name Kraton®.

Component (b) is typically present in the composition of the instant invention in an amount ranging from 10-70% by weight.

The compatibilizer (component (c)) is chosen according to the nature of component (b). It will have a backbone that is identical to component (b) and a reactive group which is compatible with component (a). This changes the morphology of the composition according to the invention such that the component (b) is distributed in component (b) in the form of globules rather than laminae.

The reactive group may be a grafting monomer that is grafted to this backbone, and is or contains at least one alpha- or beta-ethylenically unsaturated carboxylic acid or anhydrides, or a derivative thereof. Examples of such carboxylic acids and anhydrides, which may be mono-, di- or polycarboxylic acids, are acrylic acid, methacrylic acid, maleic acid, fumaric acid, itaconic acid, crotonic acid, itaconic anhydride, maleic anhydride and substituted maleic anhydride e.g. dimethyl maleic anhydride. Examples of derivatives of the unsaturated acids are salts, amides, imides and esters e.g. mono- and disodium maleate, acrylamide, maleimide and diethyl fumarate.

Maleic anhydride is a preferred grafting monomer (reactive group).

Preferred backbones for component (c) include polyethylene (low density (PE-LD), linear low density (PE-LLD), high density (HDPE) and very low density (VLDPE)) and polypropylene.

The grafting of the polymers can be carried out in the melt state, in solution or in suspension as described. The melt viscosity of the grafted polymer is not restricted, however, most effective alloying is found if the melt index, measured at 2.16 kg and 190°C is between 1 and 150 g/cm$^3$. Such grafted polymers can be prepared as known in the art (cf. European Patent Application 370 735 and 370 736).

The compatibilizer is present in the composition of the instant invention in an amount ranging from 0.1 to 15% by weight, preferably between 0.1 and 3%.

In addition to the above components, compositions according to the invention may contain conventional additives, such as melt flow modifying agents (e.g. calcium carbonate, silicas, clay, talc) and stabilizers, such as antioxidants and ultraviolet absorbers. The properties these additives bring to the composition and the amounts in which they can be used are known to the skilled artisan.

The compositions according to the invention can be prepared according to methods and techniques known in the art, e.g. by (physical) tumble blending.

A suitable compounding technique is as follows: a polyether ester and polyethylene are compounded in a Berstorff 25 mm twin screw extruder at a temperature ranging from 200-240°C. Strands of copolymer so produced are water cooled and granulated.

The compositions are made into films using blown film processing. For example, granules produced as above are fed into an extruder having a length/diameter ratio of 27-28, a mixing head and 3-zone screw and equipped with a spiral die and a blown film unit. Films so produced have a thickness of about 50 microns.

Examples

Physical blends and compounded blends are prepared having the compositions indicated in Table 1 below. Films having a thickness of 25 μm are extruded and moisture vapor transmission rate (MTVR) is measured according to ASTM E 96 BW.

Polymer A = a block polyether ester segmented elastomer containing about, by weight, 31% dimethyl terephthalate, 9% dimethyl isophthalate, 55% ethylene oxide end-capped polypropylene glycol and 16% butane diol; melt flow index (MFI) = 10 dg/min at 190°C under 2.16 kg according to ASTM D 1238).

Polymer B = Stamylan ® 2304 (available from DSM; a low density polyethylene, density of 0.92 kg m3, MFI = 4.4 dg/min (ASTM D 1238)).

Polymer C = Fusabond ® EMB 226 D (available from the DuPont Company; backbone of PE-LLD, density of 0.92 kg/m$^3$, MFI = 4.4 dg/min (ASTM D 1238), comonomer content: 7% butene, maleic anhydride content: 1%).

Table 1

|  | Control 1 | Control 2 | Control 3 | Example 1 | Example 2 |
|---|---|---|---|---|---|
| Polymer A (parts by weight) | 100 |  | 70 | 65.8 | 65.8 |
| Polymer B (parts by weight) |  | 100 | 30 | 28.2 | 28.2 |
| Polymer C (parts by weight) |  |  |  | 6 | 6 |
| Blend type | - | - | physical | physical | compound |

Table 1   (continued)

|  | Control 1 | Control 2 | Control 3 | Example 1 | Example 2 |
|---|---|---|---|---|---|
| MTVR (g/m$^2$·day) | 2025 | 9 | 253 | 735 | 657 |

Films according to the invention demonstrate improved performance properties, such as improved seal strength. This can be demonstrated by sealing the film using a Kopp sealing machine with two sealing bars; dwell time 1 sec, pressure 0.3 MPa; and testing the seal on a Zwick tensile testing machine with 100 mm/min. cross head speed.

**Claims**

**1.**   A breathable film which is formed from a thermoplastic composition comprising:

(a) at least 25% by weight of a thermoplastic polymer selected from the group consisting of a block copolyether ester, a block copolyether amide, a polyurethane, or combinations thereof,
(b) from 10% to 70% by weight of a thermoplastic homopolymer of an alpha-olefin, a co- or terpolymer containing an alpha-olefin and one or more other monomers, or a block copolymer of a vinylarene and a conjugated diene with the proviso that said component (b) is incompatible with (a); and
(c) from 0.1% to 15% by weight of a compatibilizer which is a homo- co- or terpolymer having a backbone which is identical to component (b) and a reactive group which is compatible with component (a)

wherein

(i) said film is obtained by blown film extrusion; and
(ii) said film has a moisture vapor transmission rate (MVTR) value, as measured according to ASTM E 96 BW which is approaching that of the theoretical MVTR value calculated as follows:

$$MVTR_{theor} = (MVTR_{comp.(a)}) (weight\%_{comp.(a)}) + (MVTR_{comp(b)}) (weight\%_{comp(b)})$$

**2.**   A process for forming the breathable thermoplastic film as defined in claim 1 comprising:

(a) preparing the thermoplastic composition comprising a mixture of (a), (b) and (c) ; and
(b) extruding said composition in a blown film extruder.

**Patentansprüche**

**1.**   Atmungsfähige Folie, welche aus einer thermoplastischen Zusammensetzung, umfassend

a) mindestens 25 Gew.-% eines thermoplastischen Polymers, ausgewählt aus der Gruppe, bestehend aus einem Blockcopolyetherester, einem Blockcopolyetheramid, einem Polyurethan oder Kombinationen davon,
b) von 10 bis 70 Gew.-% eines thermoplastischen Homopolymers aus einem alpha-Olefin, eines Co- oder Terpolymers, enthaltend ein alpha-Olefin und ein oder mehrere andere Monomere, oder eines Blockcopolymers aus einem Vinylaren und einem konjugierten Dien, mit der Maßgabe, daß die Komponente (b) inkompatibel mit (a) ist; und
(c) von 0,1 bis 15 Gew.-% eines kompatibel machenden Stoffes, welcher ein Homo-, Co- oder Terpolymer mit einem Grundgerüst, welches identisch mit Komponente (b) ist, und einer reaktiven Gruppe, welche kompatibel mit Komponente (a) ist, ist, erzeugt wird, wobei

(i) die Folie durch Blasfolienextrusion erhalten wird; und

(ii) die Folie einen Wert der Durchlässigkeitsrate für Feuchtigkeitsdampf (MVTR), wie gemessen nach ASTM E 96 BW, hat, welcher sich an den des theoretischen MVTR-Werts annähert, der wie folgt berechnet wird:

$$\text{MVTR}_{\text{theor}} = (\text{MVTR}_{\text{Zus.(a)}})\,(\text{Gew.-\%}_{\text{Zus.(a)}}) + (\text{MVTR}_{\text{Zus.(b)}})\,(\text{Gew.-\%}_{\text{Zus.(b)}}).$$

2. Verfahren zur Erzeugung der atmungsfähigen- thermoplastischen Folie nach Anspruch 1, umfassend:

(a) Herstellen der thermoplastischen Zusammensetzung, umfassend ein Gemisch von (a), (b) und (c); und

(b) Extrudieren der Zusammensetzung in einem Blasfolienextruder.

**Revendications**

1. Film imper-respirant qui est formé à partir d'une composition thermoplastique comprenant:

(a) au moins 25% en poids d'un polymère thermoplastique choisi dans le groupe constitué d'un copolyéther ester séquencé, d'un copolyéther amide séquencé, d'un polyuréthane, ou de combinaisons de ceux-ci,

(b) 10% à 70% en poids d'un homopolymère thermoplastique d'une alpha-oléfine, d'un co- ou terpolymère contenant une alpha-oléfine et un ou plusieurs autres monomères, ou d'un copolymère séquencé d'un vinyl-larène et d'un diène conjugué à condition que ledit composant (b) soit incompatible avec (a); et

(c) 0,1% à 15% en poids d'un agent de compatibilisation qui est un homo- co- ou terpolymère ayant un squelette qui est identique au composant (b) et un groupe réactif qui est compatible avec le composant (a)

dans lequel

(i) ledit film est obtenu par extrusion de film soufflé; et

(ii) ledit film a une valeur d'indice de transmission de vapeur (MVTR), mesurée selon ASTM E 96 BW, qui s'approche de la valeur de MVTR théorique calculée comme suit:

$$\text{MVTR}_{\text{théor}} = (\text{MVTR}_{\text{comp.(a)}})\,(\%\ \text{poids}_{\text{comp.(a)}}) + (\text{MVTR}_{\text{comp.(b)}})\,(\%\ \text{poids}_{\text{comp.(b)}})$$

2. Procédé de formation du film thermoplastique imper-respirant selon la revendication 1 comprenant:

(a) la préparation de la composition thermoplastique comprenant un mélange de (a), de (b) et de (c); et

(b) l'extrusion de ladite composition dans une extrudeuse de film soufflé.